# EUROPÄISCHE PATENTSCHRIFT

(11) **EP 1 911 394 B1**
(45) Veröffentlichungstag und Bekanntmachung des Hinweises auf die Patenterteilung: **06.05.2009**
(21) Anmeldenummer: 06021579.5
(22) Anmeldetag: 14.10.2006
(51) Int. Cl.: A61B 5/00

(54) **Lanzette mit Kapillarkanal**
Lancet with capillar channel
Lancette avec un canal capillaire

(43) Veröffentlichungstag der Anmeldung: 16.04.2008
(73) Patentinhaber: Roche Diagnostics GmbH, 68305 Mannheim (DE); F.HOFFMANN-LA ROCHE AG, 4070 Basel (CH)
(72) Erfinder: Werner, Gerhard, 69469 Weinheim (DE); Haar, Hans-Peter, 69168 Wiesloch (DE)
(74) Vertreter: Mommer, Niels

(56) Entgegenhaltungen:
- EP-A- 1 284 121
- EP-A- 1 759 633
- WO-A-01/93930
- WO-A-95/33504
- WO-A-96/00614
- WO-A1-02/50534
- US-A- 5 801 057
- US-A1- 2002 137 998

## Beschreibung

Die Erfindung betrifft eine Lanzette mit einem Lanzettenkörper, der eine Lanzettenspitze zum Erzeugen einer Einstichwunde und einem Kapillarkanal zum Fördern von Körperflüssigkeit aus einer Einstichwunde in einer Förderrichtung aufweist. Eine derartige Lanzette ist beispielsweise aus der US 2003/0171699 A1 sowie der WO 2005/084530 A2 bekannt. Eine Lanzette mit den im Oberbegriff des Anspruch 1 angegebenen Merkmalen ist aus der US 2002/137998 sowie aus der WO 01/93930 A1 bekannt.

Unter einem Kapillarkanal wird im Rahmen der vorliegenden Anmeldung ein Kanal verstanden, der so dimensioniert ist, dass eine zu untersuchende Körperflüssigkeit von Kapillarkräften durch den Kanal bewegt wird.

Lanzetten mit Kapillarkanälen zur Entnahme von Körperflüssigkeit werden beispielsweise von Diabetikern verwendet, die mehrmals täglich ihren Blutzuckerspiegel kontrollieren müssen, indem in einem Körperteil, in der Regel einem Finger, eine kleine Einstichwunde erzeugt wird, um eine Körperflüssigkeitsprobe zu gewinnen, die mit einem Messgerät zur Bestimmung des Glukosegehalts untersucht werden kann. Lanzetten mit Kapillarkanälen haben dabei den Vorteil, dass das Erzeugen der Einstichwunde und die Entnahme einer Körperflüssigkeitsprobe In einem Schritt durchgeführt werden können, was Insbesondere für Patienten mit einer aufgrund von Alter oder Krankheit reduzierten Geschicklichkeit von großer Bedeutung ist. Insbesondere kann durch eine automatische Probenentnahme mittels des Kapillarkanals die Gefahr einer Kontamination oder fehlerhafter Messergebnisse durch unsachgemäße Handhabung einer Probe minimiert werden.

Nachteilig an bekannten Lanzetten mit Kapillarkanälen sind jedoch deren im Vergleich zu herkömmlichen Lanzetten relativ hohe Herstellungskosten.

Aufgabe der Erfindung ist es deshalb, einen Weg aufzuzeigen, wie eine Lanzette mit einem Kapillarkanal zum Fördern von Körperflüssigkeit aus einer mit der Lanzette erzeugten Einstichwunde mit geringerem Aufwand hergestellt werden kann.

Diese Aufgabe wird erfindungsgemäß durch eine Lanzette mit den im Anspruch 1 angegebenen Merkmalen gelöst.

Während der Kapillarkanal der im Stand der Technik bekannten Lanzetten als Rinne ausgebildet ist, die einen großen Fertigungsaufwand verursacht, lässt sich ein erfindungsgemäßer Schlitz insbesondere in einen Lanzettenkörper aus Stahl mit wesentlich geringerem Aufwand erzeugen, beispielsweise durch Laserstrahlschneiden, chemisches Ätzen oder Stanzen. Überraschenderweise erzeugt ein senkrecht zu der Förderrichtung vollständig durch den Lanzettenkörper hindurchgehender Schlitz eine völlig ausreichende, teilweise sogar verbesserte Förderwirkung, um eine Körperflüssigkeitsprobe aus einer mit der Lanzette erzeugten Einstichwunde zu gewinnen.

Eine erfindungsgemäße Lanzette mit einem Testbereich zum optischen Untersuchen einer mittels des Kapillarkanals geförderten Körperflüssigkeitsprobe bietet deshalb bei wesentlich geringeren Herstellungskosten dieselben Vorteile wie im Stand der Technik bekannte Lanzetten mit Kapillarkanälen. Insbesondere kann der Testbereich als Abschnitt des Schlitzes besonders einfach und kostengünstig ausgebildet werden, so dass mit einer erfindungsgemäßen Lanzette eine kostengünstige Untersuchung einer Körperflüssigkeitsprobe ermöglicht wird.

Der Testbereich einer erfindungsgemäßen Lanzette umfasst ein transparentes Bauteil, das eine Benetzungsfläche zum Benetzen mit einer zu untersuchenden Körperflüssigkeitsprobe aufweist. Beispielsweise kann der Testbereich als Küvette ausgebildet sein, wobei das transparente Bauteil eine Küvettenwand bildet, deren Innenseite die Benetzungsfläche bildet. Dabei ist es besonderes günstig als transparentes Bauteil Kunststofffolie zu verwenden und zwei parallele Küvettenwände durch Bedecken des Schlitzes mit Kunststofffolie auszubilden. Auf diese Weise kann kostengünstig eine Untersuchung einer Körperflüssigkeitsprobe in Transmission ermöglicht werden.

Als Alternative zu einer Untersuchung der Körperflüssigkeitsprobe in Transmission kann das transparente Bauteil mit abgeschrägten Seitenflächen ausgestattet werden, die zur Einkopplung und Auskopplung von Untersuchungslicht genutzt werden können, das mittels Totalreflexion an der Benetzungsfläche entlang geführt wird und dabei mit einer die Benetzungsfläche benetzenden Probe in Wechselwirkung tritt.

Mit einer erfindungsgemäßen Lanzette kann eine Körperflüssigkeitsprobe insbesondere auch reagenzfrei untersucht werden. Während die Testbereiche herkömmlicher Lanzetten häufig empfindliche Reagenzien, beispielsweise Enzyme zur photometrischen Untersuchung enthalten, die eine Sterilisation erschweren, kann eine erfindungsgemäße Lanzette mit einem reagenzfreien Testbereich ausgestattet und deshalb wesentlich einfacher sterilisiert werden, beispielsweise mittels Hitze und/oder Strahlung, insbesondere Elektronenstrahlen.

Bevorzugt sind Seitenwände des Schlitzes mit einer hydrophilen Substanz beschichtet, um die Kapillarwirkung zu erhöhen. Bei einem enzymfreien Testbereich, wie er bei einer erfindungsgemäßen Lanzette bevorzugt ist, besteht eine vorteilhaft große Freiheit bei der Auswahl hydrophiler Substanzen, da keine Wechselwirkung mit einer chemischen Nachweisreaktion zu befürchten ist.

Ein weiterer Aspekt der Erfindung, der auch selbständige Bedeutung haben kann, betrifft deshalb eine Lanzette mit einem Testbereich zum optischen Untersuchen einer Körperflüssigkeitsprobe, wobei der Testbereich eine Benetzungsfläche zum Benetzen mit der Körperflüssigkeitsprobe umfasst, dadurch gekennzeichnet, dass die Benetzungsfläche als Stirnseite eines transparenten Bauteils ausgebildet ist, das eine erste Seitenfläche zum Einkoppeln von Licht und eine zweite Seitenfläche zum Auskoppeln von Licht aufweist. Die erste und die zweite Seitenfläche können schräg zu der Stirnseite, insbesondere unter einem Winkel von 45° orientiert sein, oder beispielsweise als Mantelflächen von Viertelzylindern ausgebildet sein.

Zu der ersten Seitenfläche des transparenten Bauteils eingekoppeltes Licht tritt mit einer die Stirnseite des transparenten Bauteils benetzenden Körperflüssigkeitsprobe in Wechselwirkung und wird mittels Totalreflexion zu der zweiten Seitenfläche geleitet, an der es ausgekoppelt wird, so dass es einem Detektor zugeführt werden kann.

Der Vorteil einer erfindungsgemäßen Lanzette mit einer derartigen Benetzungsfläche liegt insbesondere darin, dass wegen der Totalreflexion nur eine dünne Schicht der Körperflüssigkeitsprobe an der Stirnseite mit dem Licht in Wechselwirkung tritt und folglich bereits ein sehr kleines Probenvolumen zur Bestimmung einer Analytkonzentration, beispielsweise der Glukosekonzentration, ausreicht. Zur reagenzfreien Bestimmung einer Analytkonzentration kann beispielsweise die Absorptionscharakteristik des Analyten im mittleren infraroten Spektralbereich verwendet werden. Einzelheiten zur reagenzfreien Bestimmung einer Analytkonzentration mittels Infrarotspektroskopie sind beispielsweise in E. Diessel et al., Applied Spectroksopy, 2004, 442 bis 450 beschrieben, die diesbezüglich durch Bezugnahme zum Gegenstand der vorliegenden Anmeldung gemacht wird. Selbstverständlich ist das Material des transparenten Bauteils entsprechend dem verwendeten Spektralbereich des zur optischen Untersuchung der Körperflüssigkeit verwendeten Lichts zu wählen, so dass das transparente Bauteil für dieses Licht hinreichend transparent ist. Geeignete Materialien, die im mittleren infraroten Spektralbereich, dem nahen infraroten Spektralbereich und/oder dem sichtbaren Spektralbereich ausreichend transparent sind, sind insbesondere Glas und Kunststoffe, insbesondere Polyethylen und Polycarbonat.

Weitere Einzelheiten und Vorteile der Erfindung werden anhand von Ausführungsbeispielen unter Bezugnahme auf die beigefügten Zeichnungen erläutert, wobei gleiche und einander entsprechende Bauteile mit identischen Bezugszeichen gekennzeichnet sind. Die dabei erläuterten Merkmale können einzeln oder gesondert zum Gegenstand von Ansprüchen gemacht werden. Es zeigen:
- Fig. 1: ein Ausführungsbeispiel einer erfindungsgemäßen Lanzette in einer Explosionsdarstellung;
- Fig. 2: das in Fig. 1 dargestellte Ausführungsbeispiel in einer Schrägansicht;
- Fig. 3: das in Fig. 1 dargestellte Ausführungsbeispiel in einer Draufsicht;
- Fig. 4: das in Fig. 1 dargestellte Ausführungsbeispiel in einer Frontansicht;
- Fig. 5: das in Fig. 1 dargestellte Ausführungsbeispiel in einer Rückansicht;
- Fig. 6: das in Fig. 1 dargestellte Ausführungsbeispiel in einem Längsschnitt entlang des Kapillarkanals;
- Fig. 7: eine Detailansicht zu Fig. 6;
- Fig. 8: den Lanzettenkörper mit der Lanzettenspitze des in Fig. 1 dargestellten Ausführungsbeispiels in einer Schrägansicht;
- Fig. 9: den in Fig. 8 dargestellten Lanzettenkörper in einer Draufsicht;
- Fig. 10.: eine Frontansicht zu Fig. 8;
- Fig. 11: eine Rückansicht zu Fig. 8;
- Fig. 12: den in Fig. 8 dargestellten Lanzettenkörper in einem Längsschnitt entlang des Kapillarkanals;
- Fig. 13: ein weiteres Ausführungsbeispiel einer erfindungsgemäßen Lanzette;
- Fig. 14: ein Ausführungsbeispiel eines Messgeräts zur Bestimmung einer Analytkonzentration einer Körperflüssigkeitsprobe in dem Testbereich der in Fig. 13 dargestellten Lanzette;
- Fig. 15: das in Fig. 12 dargestellte Messgerät in einer Schnittansicht;
- Fig. 16: ein weiteres Ausführungsbeispiel einer erfindungsgemäßen Lanzetten in einer Explosionsdarstellung;
- Fig. 17: das in Fig. 16 dargestellte Ausführungsbeispiel in einer Schrägansicht;
- Fig. 18: das in Fig. 16 dargestellte Ausführungsbeispiel in einer Draufsicht;
- Fig. 19: das in Fig. 16 dargestellte Ausführungsbeispiel in einer Frontansicht;
- Fig. 20: das in Fig. 16 dargestellte Ausführungsbeispiel in einer Rückansicht;
- Fig. 21: das in Fig. 16 dargestellte Ausführungsbeispiel in einem Längsschnitt entlang des Kapillarkanals;
- Fig. 22: eine Detailansicht zu Fig. 21;
- Fig. 23: eine Detailansicht zu Fig. 21 eines weiteren Ausführungsbeispiels; und
- Fig. 24: ein schematisches Ausführungsbeispiel eines Stechsystems umfassend eine Lanzette und ein Stechgerät.

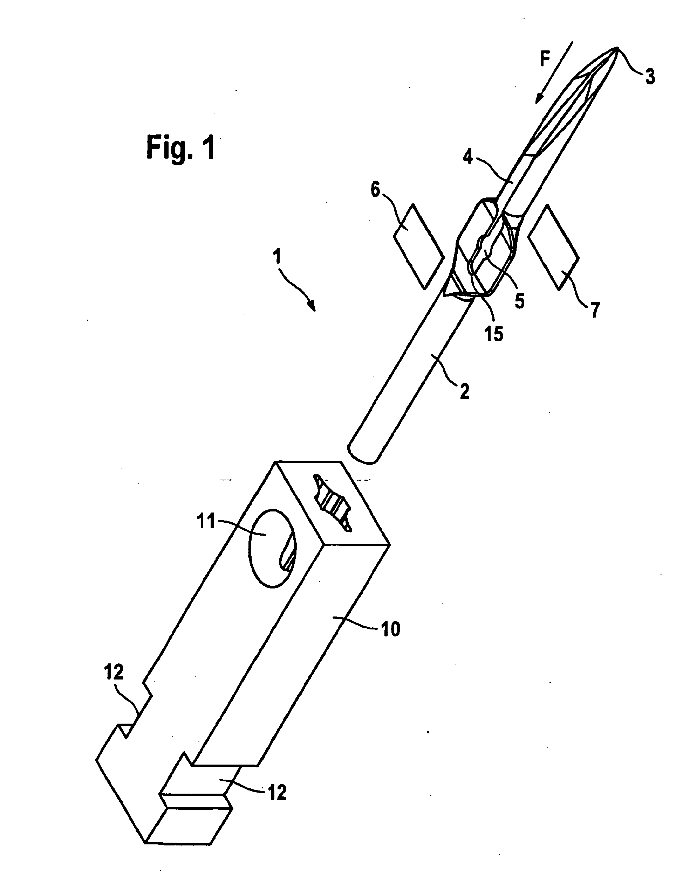

Die in den Figuren 1 bis 3 dargestellte Lanzette 1 umfasst einen Lanzettenkörper 2 aus Stahl mit einer Lanzettenspitze 3 und einem von der Lanzettenspitze 3 ausgehenden Kapillarkanal 4 zum Fördern von Körperflüssigkeit aus einer mit der Lanzette erzeugten Einstichwunde in einer Förderrichtung F. Die Förderrichtung F ist naturgemäß durch die Richtung des Kapillarkanals 4 vorgegeben. Der Kapillarkanal 4 führt bei dem dargestellten Ausführungsbeispiel zu einem enzymfreien Testbereich 5 zum optischen Untersuchen einer mittels des Kapillarkanals 4 geförderten Körperflüssigkeitsprobe. Der Testbereich 5 ist mit Kunststofffolie 6, 7, beispielsweise aus Polyethylen bedeckt. Der Lanzettenkörper 2 ist in einem den Testbereich 5 umgebenden Abschnitt flach ausgebildet. Bei dem dargestellten Ausführungsbeispiel ist der Lanzettenkörper im Übrigen zylindrisch geformt, jedoch kann der Lanzettenkörper auch vollständig flach ausgebildet sein und beispielsweise aus Stahlblech ausgestanzt oder mit Laserschneiden ausgeschnitten sein.

Bei dem dargestellten Ausführungsbeispiel ist der Testbereich 5 zwischen zwei gegenüberliegenden Wänden aus Kunststofffolie 6, 7 ausgebildet, die bevorzugt parallel verlaufen. Auf diese Weise lässt sich mit einfachen Mitteln eine Küvette bilden, in der Körperflüssigkeit in Transmission optisch untersucht werden kann.

Damit sich der Testbereich 5 leichter mit Körperflüssigkeit füllt, ist es günstig, den Testbereich 5 mit einer Entlüftungsöffnung 15 zu versehen, um ein Ausströmen von Gas aus dem Testbereich 5 zu ermöglichen. Eine Entlüftungsöffnung kann beispielsweise in der Weise ausgebildet sein, dass an den Testbereich 5 auf der von der Spitze 3 abgewandten Seite ein Schlitzabschnitt abgrenzt, der zumindest auf einer Seite teilweise offen ist, also nicht mit Kunststofffolie bedeckt ist.

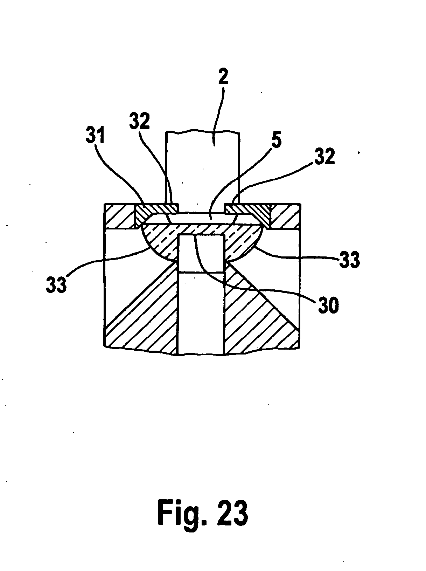

Der Lanzettenkörper 2 steckt in einem Halter 10 aus Kunststoff, der gegenüberliegende Öffnungen 11 für eine optische Untersuchung von Körperflüssigkeit in dem Testbereich 5 aufweist. Ferner hat der Halter 10 Kopplungselemente 12, die bei dem dargestellten Ausführungsbeispiel als Ausnehmungen ausgebildet sind, mit denen die Lanzette 1 an einen Lanzettenantrieb eines schematisch in Figur 23 dargestellten Stechgeräts ankoppeln kann.

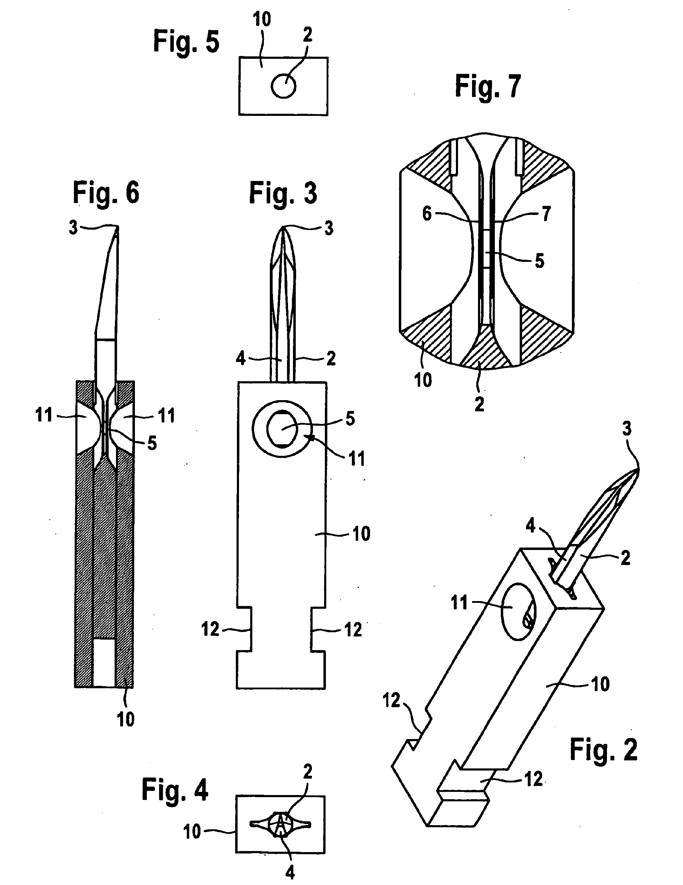

Fig. 4 zeigt die anhand der Figuren 1 bis 3 beschriebene Lanzette 1 in einer Vorderansicht, d.h. mit Blick auf die Spitze 3. In entsprechender Weise zeigt Fig. 5 die Lanzette 1 in einer Rückansicht.

Der Kapillarkanal 4 der Lanzette 1 ist von einem Schlitz gebildet, der senkrecht zu der Förderrichtung F vollständig durch den Lanzettenkörper 2 hindurchgeht. Die Seitenwände des Schlitzes 4 sind mit einer hydrophilen Substanz, beispielsweise Sorbat, bedeckt, um die Kapillarwirkung zu erhöhen.

In Fig. 6 ist ein Längsschnitt durch die Lanzette 1 entlang des Kapillarkanals 4 dargestellt. Der Testbereich 5 ist dabei als ein Abschnitt des den Kapillarkanal 4 bildenden Schlitzes ausgebildet. Der Schlitz 4 ist deshalb in dem den Testbereich 5 bildenden Abschnitt breiter als an der Spitze 3. Bei dem dargestellten Ausführungsbeispiel geht der Kapillarkanal 4 zwar von der Lanzettenspitze 3 aus, jedoch ist ein kleiner Bereich der Lanzettenspitze 3 selbst nicht geschlitzt, damit eine größere mechanische Stabilität und Schärfe für einen schmerzarmen Einstichvorgang ermöglicht wird. Selbstverständlich ist es aber auch möglich, den Schlitz 4 auch durch den vordersten Bereich der Lanzettenspitze 3 hindurchzuführen und vorne aus der Lanzettenspitze austreten zu lassen.

In Fig. 7 ist eine Detailansicht zu Fig. 6 gezeigt, in welcher der Testbereich 5 mit dem ihn umgebenden flachen Abschnitt des Lanzettenkörpers 2 sowie den den Testbereich 5 bedeckenden transparenten Kunststofffolien 6, 7 dargestellt ist.

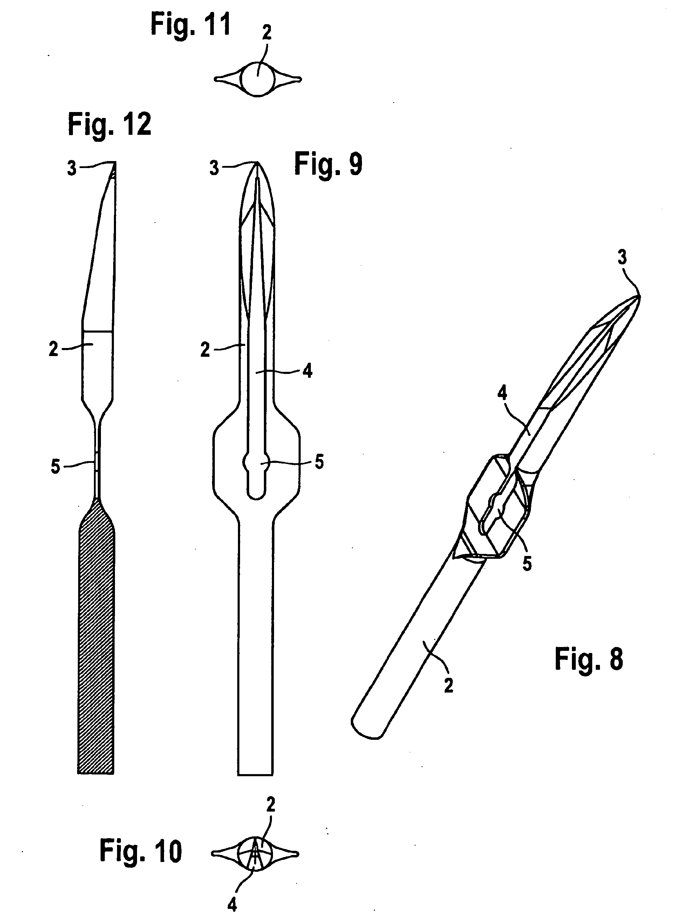

Der Lanzettenkörper 2 mit der Lanzettenspitze 3 ist in den Figuren 8 bis 12 dargestellt. Dabei zeigt Fig. 8 eine Schrägansicht des Lanzettenkörpers 2, Fig. 9 eine Draufsicht, Fig. 10 eine Frontansicht mit Blick auf die Lanzettenspitze, Fig. 11 eine Rückansicht und Fig. 12 einen Längsschnitt entlang des Kapillarkanals 4. Anhand dieser Figuren ist ersichtlich, dass der Lanzettenkörper 2 in einem den Testbereich 5 umgebenden Abschnitt flach und in einem davor und dahinter liegenden Abschnitt zylindrisch ausgebildet ist. Der vordere zylindrische Abschnitt des Lanzettenkörpers 2 ist dabei schräg angeschnitten, um eine scharfe Spitze 3 auszubilden, die einen möglichst schmerzarmen Einstich in Körpergewebe ermöglicht.

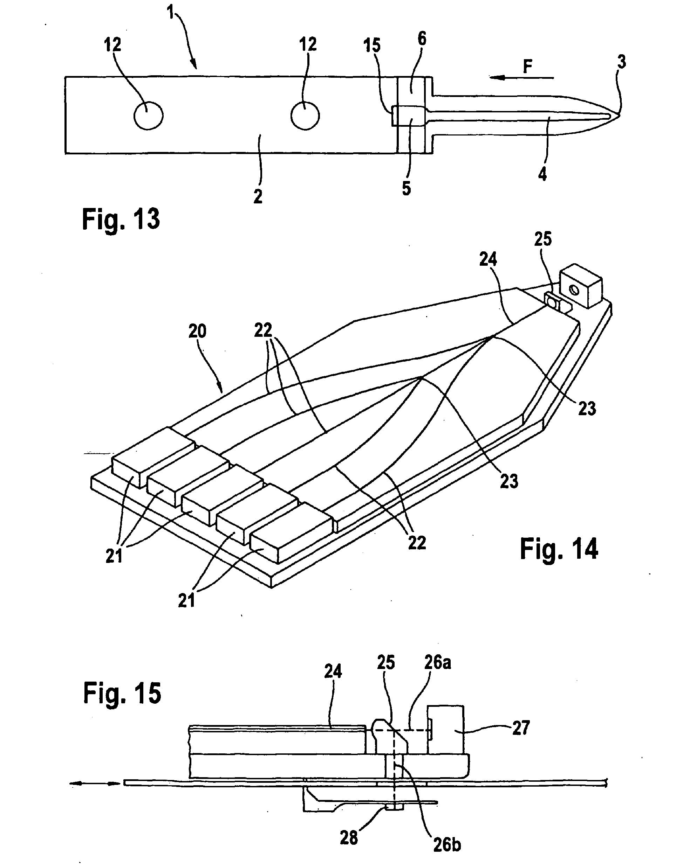

In Fig. 13 ist ein weiteres Ausführungsbeispiel einer Lanzette 1 dargestellt. Diese Lanzette 1 umfasst ebenfalls einen Lanzettenkörper 2 aus Stahl mit einer Lanzettenspitze 3 zum Erzeugen einer Einstichwunde und einem Kapillarkanal 4 zum Fördern von Körperflüssigkeit aus einer Einstichwunde in einer Förderrichtung F. Der Kapillarkanal 4 ist ebenso wie bei dem im vorhergehenden erläuterten Ausführungsbeispiel von einem Schlitz gebildet, der durch den Lanzettenkörper 2 senkrecht zu der Förderrichtung F vollständig hindurchgeht. Bei dem in Fig. 11 dargestellten Ausführungsbeispiel handelt es sich um eine Flachlanzette, da der Lanzettenkörper 2 aus einem Metallband ausgeschnitten ist, was beispielsweise durch Ausstanzen oder Laserschneiden kostengünstig möglich ist. Der Kapillarkanal 4 einschließlich des Testbereichs 5 sowie Ausnehmungen als Kopplungselement 12 zum Ankoppeln der Lanzette 1 an einen Antrieb eines Stechgeräts können beim Ausscbneiden des Lanzettenkörpers 2 aus einem Metallband ebenfalls mit geringem Aufwand hergestellt werden. Der den Kapillarkanal bildende Spalt 4 erstreckt sich geradlinig von dem Bereich der Lanzettenspitze 3 bis zu einem verbreiterten Abschnitt, der den Testbereich 5 bildet. Der Testbereich 5 ist zwischen zwei transparenten Kunststofffolien 6 gebildet, so dass Küvettenwände für eine transmissionsoptische Untersuchung einer Körperflüssigkeitsprobe in dem Testbereich 5 vorhanden sind. Der Testbereich 5 hat eine Entlüftungsöffnung 15, durch die beim Einströmen von Körperflüssigkeit in den Testbereich 5 Gas ausströmen kann, so dass sich der Testbereich 5 leichter mit Körperflüssigkeit füllt.

Weitere Kosteneinsparungen können dadurch erzielt werden, dass die Kunststofffolie 6, mit welcher der Testbereich 5 abgedeckt ist, Teil einer größeren Folie ist, mit der die gesamte Lanzette 1 zur Sterilverpackung umhüllt ist. Diese größere Folie ist mit dem Lanzettenkörper 2 in dem in Fig. 13 dargestellten streifenförmigen Bereich verklebt, so dass bei einem Aufreisen der Sterilverpackung im Bereich der Lanzettenspitze 3 die Küvettenwände 6 intakt bleiben.

Der Testbereich 5 kann Nachweisreagenzien zum photometrischen Nachweis eines Analyten, beispielsweise Glukose, enthalten. Geeignete Nachweisreagenzien sind bei Teststreifen zur photometrischen Glukosebestimmung seit langem gebräuchlich und bedürfen deshalb an dieser Stelle keiner näheren Erläuterung. Bei Anwesenheit von Glukose bewirken derartige Nachweisreagenzien eine Verfärbung, die photometrisch zur Konzentrationsbestimmung ausgewertet werden kann. Bei dem in Fig. 13 dargestellten Ausführungsbeispiel ist der Testbereich 5 jedoch frei von Nachweisreagenzien. Die Bestimmung einer Analytkonzentration erfolgt durch eine Transmissionsmessung bei mehreren, beispielsweise fünf, Wellenlängen, in denen der Analyt ein charakteristisches Absorptionsverhalten zeigt. Beispielsweise enthält das Absorptionsspektrum von Glukose in dem Wellenlängenbereich von 5 µm bis 15 µm einige charakteristische Absorptionsbereiche, die zur Identifikation und Konzentrationsbestimmung genutzt werden können.

Fig. 15 zeigt schematisch ein Messgerät 20, mit dem eine Analytkonzentration in einer Körperflüssigkeit in dem Testbereich 5 des in Fig. 13 dargestellten Ausführungsbeispiels bestimmt werden kann. Das Messgerät 20 umfasst mehrere Strahlungsquellen 21, mit denen jeweils schmalbandiges Licht einer Wellenlänge erzeugt werden kann, in der der Analyt ein charakteristisches Absorptionsverhalten zeigt. Bei dem dargestellten Ausführungsbeispiel handelt es sich bei den Strahlungsquellen 21 um MIR-LEDs, vorzugsweise um Kaskaden-Halbleiter-Laser, mit denen Laserstrahlung im mittleren infraroten Spektralbereich erzeugt werden kann. Das von den Strahlungsquellen 21 erzeugte Laserlicht des mittleren infraroten Spektralbereichs wird mittels Lichtleitern 22 geleitet und mittels Beam-Combinern 23 in einem einzigen Lichtleiter 24 zusammengeführt. Mit dem Lichtleiter 24 wird die Infrarotstrahlung der Lichtquellen 21 einem Strahlteiler 25 zugeführt, der zwei Teilstrahlen 26a und 26b erzeugt, die in der in Fig. 15 dargestellten Querschnittsansicht zu Fig. 14 dargestellt sind. Ein Teilstrahl 26a wird als Referenzstrahl einem Detektor 27 zugeführt. Der andere Teilstrahl 26b wird durch den Testbereich 5 und eine darin enthaltene Flüssigkeitsprobe hindurch zu einem spektralsensitiven Detektor 28 geführt. Durch Vergleich der Signale der beiden Detektoren 27 und 28 für die einzelnen Wellenlängen der Strahlungsquellen 21 lässt sich feststellen, welcher Anteil der jeweiligen Wellenlänge absorbiert wurde. Aus diesem Anteil kann auf die Analytkonzentration der in dem Testbereich 5 enthaltenen Körperflüssigkeitsprobe geschlossen werden.

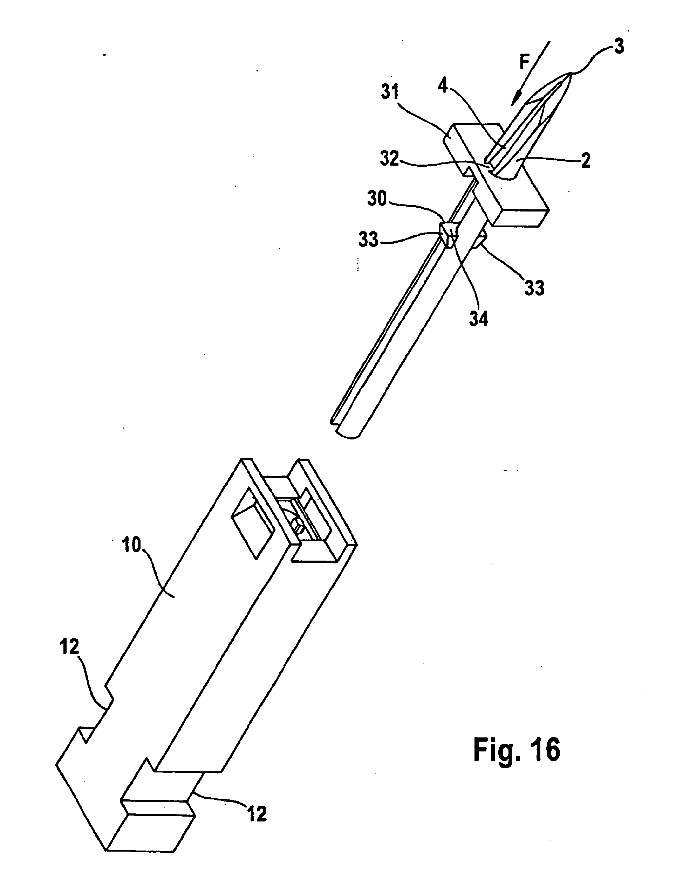

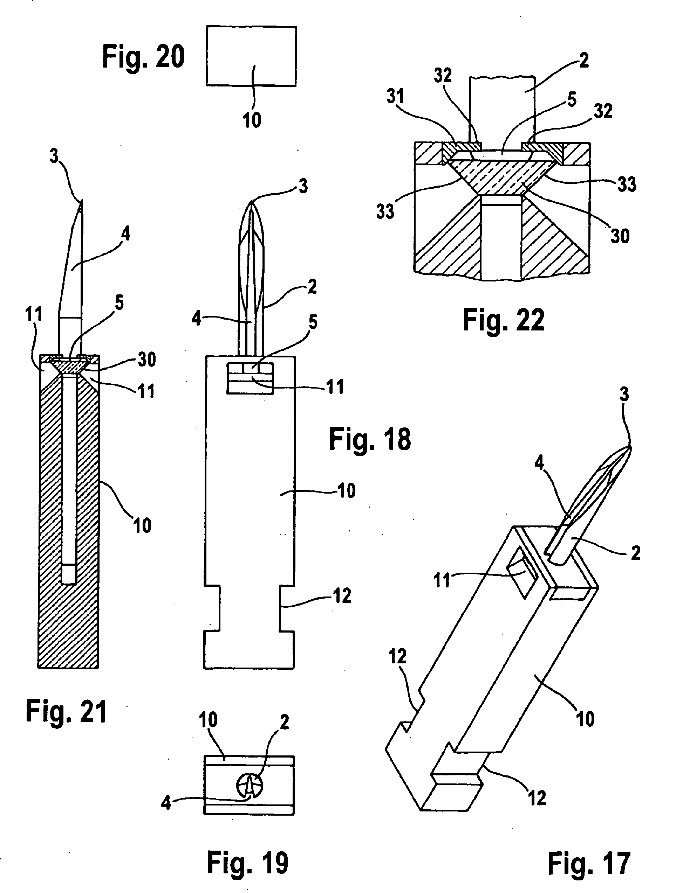

In den Figuren 16 bis 18 ist ein weiteres Ausführungsbeispiel einer erfindungsgemäßen Lanzette mit einem von der Lanzettenspitze 3 ausgehenden Kapillarkanal 4 dargestellt. Dieses Ausführungsbeispiel unterscheidet sich von dem in den Figuren 1 bis 3 dargestellten Ausführungsbeispiel im wesentlichen durch die Ausbildung des Testbereichs 5. Der Testbereich 5 ist in Fig. 21 dargestellt, die einen Schnitt durch die Lanzette 1 entlang des Kapillarkanals 4 zeigt, und insbesondere in Fig. 22 zu erkennen, die eine Detailansicht von Fig. 21 zeigt.

Der Testbereich 5 wird von einem Abschnitt des den Kapillarkanal 4 bildenden Schlitzes gebildet, der durch den Lanzettenkörper 2 senkrecht zu der Förderrichtung F vollständig hindurchgeht. Der Testbereich 5 ist auf einer Seite durch die Stirnseite des durch den Schlitz 4 hindurchgeschobene transparenten Bauteils 30 und auf der anderen Seite durch eine auf den Lanzettenkörper 2 aufgeschobene Kappe 31 begrenzt. Die Kappe 31 weist zwei gegenüberliegende Nasen 32 auf, die ein Stück weit in den Kapillarkanal 4 hineinragen, so dass zwischen den beiden Nasen 32 ein Abstand frei bleibt, durch den eine Körperflüssigkeitsprobe in den Testbereich 5 gelangen kann.

Das transparente Bauteil 30 des in Figur 22 dargestellten Ausführungsbeispiels weist zwei gegenüberliegende Seitenflächen 33 auf, die unter einem Winkel von 45°, gemessen zu der dem Testbereich 5 zugewandten Stirnseite 34, abgeschrägt sind. Die Stirnseite 34 des Trägers 30 bildet eine Benetzungsfläche, die zum Untersuchen einer Körperflüssigkeitsprobe mit der Körperflüssigkeitsprobe benetzt wird. Wird Licht zur Untersuchung einer Probe in dem Testbereich 5 durch die Öffnung 11 des Halters 10 auf die darunter liegende erste Seitenfläche 33 gestrahlt, so wird dieses Licht in einem Grenzbereich zwischen der Stirnseite 34 des transparenten Bauteils 30 und einer es benetzenden Körperflüssigkeitsprobe mehrfach total reflektiert, bis es an der zweiten Seitenfläche 33 austritt und einem Detektor zugeführt werden kann. Diese Messprinzip wird als "Attenuated Total Reflection" bezeichnet und ist beispielsweise in Applied Optics, Band 42, Seiten 745 bis 749 von Y. Kim et al. beschrieben. Hinsichtlich Details zur Durchführung von ATR-Messungen wird diese Veröffentlichung durch Bezugnahme zum Gegenstand der vorliegenden Anmeldung gemacht.

Wie in Figur 23 dargestellt ist, können die gegenüberliegenden Seitenflächen 33 auch als Mantelflächen von Viertelzylindern ausgeformt sein. Das in Figur 23 dargestellte Ausführungsbeispiel hat den Vorteil einer besonders robusten Ein- und Ausstrahlgeometrie.

Das transparente Bauteil 30 ist bevorzugt aus Kunststoff, insbesondere aus Polyethylen oder Polycarbonat. Die erste Seitenfläche 33 zum Einkoppeln von Licht und die zweite Seitenfläche 33 zum Auskoppeln von Licht sind schräg zu der Stirnseite 30 orientiert und von dem Testbereich 5 abgewandt. Die beiden Seitenflächen 33 werden deshalb bei der Untersuchung einer Körperflüssigkeitsprobe nicht benetzt.

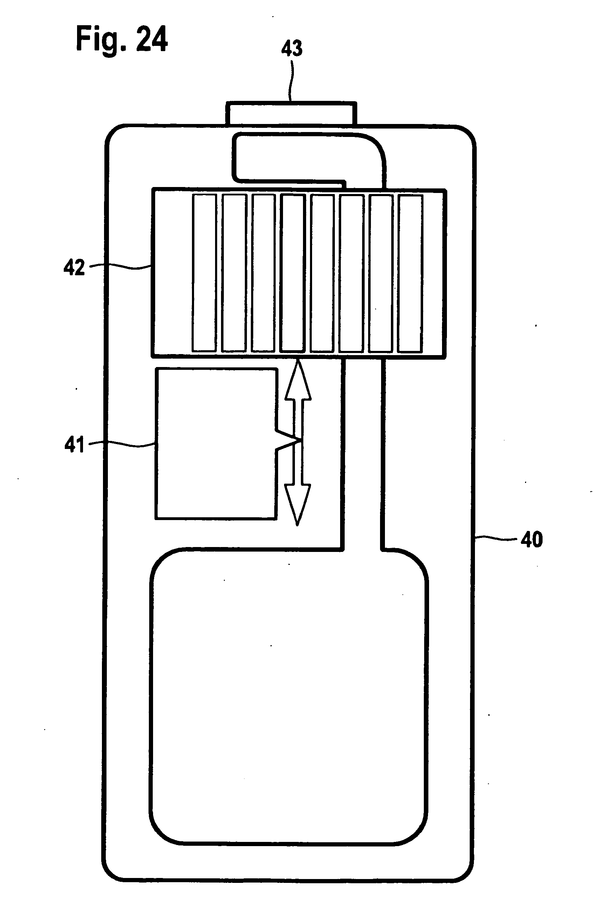

In Fig. 24 ist schematisch ein Stechgerät 40 dargestellt, dass mit der beschriebenen Lanzette 1 ein Stechsystem zum Erzeugen einer Einstichwunde bildet. Das Stechgerät 40 hat ein Anpressteil 40, mit dem es an ein Körperteil eines Benutzers gepresst werden kann, in dem eine Einstichwunde erzeugt werden soll. Bis zum Gebrauch lagern die Lanzetten 1 in dem Stechgerät 40 in einem Magazin 42. Das dargestellte Stechgerät 40 hat einen schematisch dargestellten Lanzettenantrieb 41 zum Bewegen einer in das Stechgerät 40 eingesetzten Lanzette 1 für eine Einstichbewegung, eine Messeinrichtung 20 umfassend eine Lichtquelle und einen Detektor zum optischen Untersuchen einer Körperflüssigkeitsprobe in dem Testbereich 5 einer in die Stechvorrichtung 40 eingesetzten Lanzette 1.

Das Stechgerät 40 und die Lanzetten 1 sind derart ausgebildet, dass zum optischen Untersuchen einer Körperflüssigkeitsprobe in dem Testbereich 5 einer in die Stechvorrichtung 40 eingesetzten Lanzette 1 von der Lichtquelle ausgesandtes Licht auf die in Figur 22 dargestellte Seitenfläche 33 eines den Testbereich der Lanzette mit einer Benetzungsfläche begrenzenden transparenten Bauteils 30 trifft und durch mehrfache Totalreflexion durch ein Grenzbereich zwischen der Benetzungsfläche 34 und einer die Benetzungsfläche benetzenden Körperflüssigkeitsprobe zu dem Detektor geleitet wird.

## Patentansprüche

1. Lanzette mit
einem Lanzettenkörper (2), der eine Lanzettenspitze (3) zum Erzeugen einer Einstichwunde und einen Kapillarkanal (4) zum Fördern von Körperflüssigkeit aus einer Einstichwunde in einer Förderrichtung (F) aufweist,
einem Testbereich (5) zum optischen Untersuchen einer mittels des Kapillarkanals (4) geförderten Körperflüssigkeitsprobe, wobei der Testbereich (5) ein transparentes Bauteil (6,7,30) umfasst, das eine Benetzungsfläche zum Benetzen mit einer zu untersuchenden Körperfilüssigkeitsprobe aufweist,
**dadurch gekennzeichnet, dass**
der Kapillarkanal (4) von einem Schlitz gebildet ist, der durch den Lanzettenkörper (2) senkrecht zu der Förderrichtung (F) vollständig hindurchgeht, und dass
der Testbereich (5) als ein Abschnitt des Schlitzes (4) ausgebildet ist.

2. Lanzette nach Anspruch 1, **dadurch gekennzeichnet, dass** das transparente Bauteil (6, 7) eine Küvettenwand des als Küvette ausgebildeten Testbereich (5) ist, wobei eine Innenseite der Küvettenwand (6, 7) die Benetzungsfläche bildet.

3. Lanzette nach Anspruch 2, **dadurch gekennzeichnet, dass** das transparente Bauteil (6, 7) eine Kunststofffolie (6, 7) ist.

4. Lanzette nach einem Anspruch 3, **dadurch gekennzeichnet, dass** zwei gegenüberliegende Küvettenwände aus Kunststofffolle (6, 7) sind, die den Schlitz (4) bedeckt.

5. Lanzette nach einem der vorhergehenden Ansprüche, **dadurch gekennzeichnet, dass** das transparente Bauteil (30) eine die Benetzungsfläche (34) bildende Stirnseite hat und eine erste Seitenfläche (33) zum Einkoppeln von Licht und eine zweite Seitenfläche (33) zum Auskoppeln von Licht aufweist.

6. Lanzette nach Anspruch 5, **dadurch gekennzeichnet, dass** die erste Seitenfläche (33) und die zweite Seitenfläche (33) schräg zur Stirnseite orientiert sind, insbesondere unter einem Winkel von 45° zu der Stirnseite (34) orientiert sind.

7. Lanzette nach Anspruch 6, **dadurch gekennzeichnet, dass** die erste Seitenfläche (33) und die, zweite Seitenfläche (33) jeweils als Mantelfläche eines Viertelzylinders ausgebildet sind.

8. Lanzette nach einem der Ansprüche 3 bis 7, **dadurch gekennzeichnet, dass** der Schlitz (4) in dem den Testbereich (5) bildenden Abschnitt breiter als an der Spitze (3) ist.

9. Lanzette nach einem der Ansprüche 2 bis 8, **dadurch gekennzeichnet, dass** der Testbereich (5) eine Entlüftungsöffnung (15) aufweist, um ein Ausströmen von Gas aus dem Testbereich (5) zu ermöglichen.

10. Lanzette nach einem der vorhergehenden Ansprüche, **dadurch gekennzeichnet, dass** der Testbereich (5) enzymfrei ist.

11. Lanzette nach einem der vorhergehenden Ansprüche, **dadurch gekennzeichnet, dass** der den Kapillarkanal bildende Schlitz (4) zumindest auf einem Abschnitt eine mit einer hydrophilen Substanz bedeckte Seitenwand hat.

12. Stechsystem zum Erzeugen einer Einstichwunde umfassend eine Lanzette (1) nach einem der vorhergehenden Ansprüche und ein Stechgerät (40) mit
einem Lanzettenantrieb zum Bewegen einer in das Stechgerät eingesetzten Lanzette (1) für eine Einstichbewegung,
einer Lichtquelle (21) und einen Detektor (27, 28) zum optischen Untersuchen einer Körperflüssigkeitsprobe in dem Testbereich (5) einer in die Stechvorrichtung eingesetzten Lanzette (1),
**dadurch gekennzeichnet, dass**
die Lanzette einen Testbereich (5) zum optischen Untersuchen einer Körperflüssigkeitsprobe aufweist, wobei der Testbereich (5) ein transparentes Bauteil (30) umfasst, das eine Stirnseite als Benetzungsfläche (34) zum Benetzen mit der zu untersuchenden Körperflüssigkeitsprobe, eine erste Seitenfläche (33) zum Einkoppeln von Licht und eine zweite Seitenfläche (33) zum Auskoppeln von Licht aufweist, wobei die erste Seitenfläche (33) und die zweite Seitenfläche (33) schräg zu der Stirnseite orientiert sind, und wobei das Stechgerät (40) und die Lanzette (1) derart ausgebildet sind, dass zum optischen Untersuchen einer die Benetzungsfläche (34) benetzenden Körperflüssigkeitsprobe einer in das Stechgerät eingesetzten Lanzette (1) von der Lichtquelle (21) ausgesandtes Licht auf die Schrägfläche (33) des transparenten Bauteils (30) der eingesetzten Lanzette (1) trifft und durch Totalreflexion durch einen Grenzbereich zwischen der Benetzungsfläche (34) und der die Benetzungsfläche (34) benetzenden Körperflüssigkeitsprobe zu dem Detektor (28) geleitet wird.

## Claims

1. Lancet comprising
a lancet body (2) having a lancet tip (3) for creating a puncture wound and a capillary channel (4) to convey body fluid from a puncture wound in a conveyance direction (F),
a test area (5) for optical examination of a body fluid sample conveyed by means of the capillary channel (4), wherein the test area (5) comprises a transparent component (6, 7, 30) that has a wetting surface for wetting with a body fluid sample to be examined,
**characterized in that**
the capillary channel (4) is formed by a slot that extends perpendicular to the conveyance direction (F) through the entire lancet body (2), and the test area (5) is configured as a section of the slot (4).

2. Lancet according to claim 1, **characterized in that** the transparent component (6, 7) is a cuvette wall of the test area (5) configured as a cuvette, whereby an inside of the cuvette wall (6, 7) constitutes the wetting surface.

3. Lancet according to claim 2, **characterized in that** the transparent component (6, 7) is a plastic sheet (6, 7).

4. Lancet according to claim 3, **characterized in that** two opposite cuvette walls are made out of plastic sheet (6, 7) that covers the slot (4).

5. Lancet according to any of above claims, **characterized in that** the transparent component (30) has a front side constituting the wetting surface (34) and a first lateral face (33) for coupling of incoming light and a second lateral face (33) for decoupling of outgoing light.

6. Lancet according to claim 5, **characterized in that** the first lateral face (33) and the second lateral face (33) are obliquely set to the front side, in particular at an angle of 45° to the front side (34).

7. Lancet according to claim 6, **characterized in that** both the first lateral face (33) and the second lateral face (33) are configured as the surface area of a quarter cylinder.

8. Lancet according to any of the claims 3 to 7, **characterized in that** the slot (4) is wider in the section constituting the test area (5) than at the tip (3).

9. Lancet according to any of the claims 2 to 8, **characterized in that** the test area (5) is provided with a vent hole (15) in order to facilitate the exhaust of gas from the test area (5).

10. Lancet according to any of above claims, **characterized in that** the test area (5) is enzyme-free.

11. Lancet according to any of above claims, **characterized in that** the slot (4) constituting the capillary channel (4) has at least at one section a lateral face coated with a hydrophilic substance.

12. Pricking system for creating a puncture wound comprising a lancet (1) according to any of above claims and a pricking device (40) with
a lancet drive for the puncturing movement of a lancet inserted in the pricking device,
a light source (21) and a detector (27, 28) for the optical examination of a body fluid sample in the test area (5) of a lancet (1) inserted in the pricking device (1),
**characterized in that**
the lancet is provided with a test area (5) for the optical examination of a body fluid sample, said test area (5) comprising a transparent component (30) that has a front side as wetting surface (34) for wetting with the body fluid sample to be examined, a first lateral face (33) for coupling of incoming light and a second lateral face (33) for decoupling of outgoing light, whereby the first lateral face (33) and the second lateral face (33) are set obliquely to the front side and wherein the pricking device (40) and the lancet (1) are configured in such a manner that for the optical examination of a body fluid sample, wetting the wetting surface (34) of a lancet (1) inserted into the pricking device, light emitted from the light source (21) impinges upon the oblique face (33) of the transparent component (30) of the inserted lancet (1) and is then conveyed to the detector (28) by total internal reflection through an interface region between the wetting surface (34) and the body fluid sample wetting the wetting surface (34).

## Revendications

1. Lancette comportant :
➢ un corps (2), qui comporte une pointe (3) destinée à réaliser une incision, et un conduit capillaire (4) pour transporter le liquide corporel hors de l'incision dans un sens de transport (F),
➢ une zone de test (5) pour l'examen optique d'un échantillon de liquide corporel transporté au moyen du conduit capillaire (4), ladite zone de test (5) comportant un élément transparent (6, 7, 30), qui possède une surface de mouillage destinée à être mouillée par un échantillon de liquide corporel à examiner,
**caractérisée en ce que**
➢ le conduit capillaire (4) est formé par une fente, qui traverse complètement le corps (2) de la lancette perpendiculairement au sens de transport (F), et **en ce que**
➢ la zone de test (5) est réalisée sous la forme d'un tronçon de la fente (4).

2. Lancette selon la revendication 1, **caractérisée en ce que** l'élément transparent (6, 7) est une paroi de cuvette de la zone de test (5) réalisée sous forme de cuvette, une face intérieure de ladite paroi de cuvette (6, 7) formant la surface de mouillage.

3. Lancette selon la revendication 2, **caractérisée en ce que** l'élément transparent (6, 7) est un film plastique (6, 7).

4. Lancette selon la revendication 3, **caractérisée en ce que** deux parois de cuvette face à face sont réalisées dans un film plastique (6, 7), qui recouvre la fente (4).

5. Lancette selon l'une quelconque des revendications précédentes, **caractérisée en ce que** l'élément transparent (30) possède une face frontale formant la surface de mouillage (34) et comporte une première surface latérale (33) pour l'introduction de la lumière et une deuxième surface latérale (33) pour la sortie de la lumière.

6. Lancette selon la revendication 5, **caractérisée en ce que** la première surface latérale (33) et la deuxième surface latérale (33) sont orientées en oblique par rapport à la face frontale, orientées en particulier en formant un angle de 45° avec la face frontale (34).

7. Lancette selon la revendication 6, **caractérisée en ce que** la première surface latérale (33) et la deuxième surface latérale (33) sont réalisées respectivement comme paroi latérale d'un quart de cylindre.

8. Lancette selon l'une quelconque des revendications 3 à 7, **caractérisée en ce que** la fente (4), dans le tronçon formant la zone de test (5), est plus large qu'au niveau de la pointe (3).

9. Lancette selon l'une quelconque des revendications 2 à 8, **caractérisée en ce que** la zone de test (5) comporte une ouverture de purge d'air (15) pour permettre l'évacuation de gaz hors de la zone de test (5).

10. Lancette selon l'une quelconque des revendications précédentes, **caractérisée en ce que** la zone de test (5) est exempte d'enzymes.

11. Lancette selon l'une quelconque des revendications précédentes, **caractérisée en ce que** la fente (4), formant le conduit capillaire, comporte au moins sur un tronçon une paroi latérale recouverte d'une substance hydrophile.

12. Système autopiqueur destiné à réaliser une incision, comportant une lancette (1) selon l'une quelconque des revendications précédentes et un autopiqueur (40) comportant :
➢ un actionneur de lancette pour déplacer une lancette (1), insérée dans l'autopiqueur, pour effectuer un mouvement de piqûre,
➢ une source lumineuse (21) et un détecteur (27, 28) pour l'examen optique d'un échantillon de liquide corporel dans la zone de test (5) d'une lancette (1) insérée dans l'autopiqueur,
**caractérisé en ce que**
➢ la lancette comporte une zone de test (5) pour l'examen optique d'un échantillon de liquide corporel, ladite zone de test (5) comportant un élément transparent (30), qui possède une face frontale en tant que surface de mouillage destinée à être mouillée par un échantillon de liquide corporel à examiner, une première surface latérale (33) pour l'introduction de la lumière et une deuxième surface latérale (33) pour la sortie de la lumière, la première surface latérale (33) et la deuxième surface latérale (33) étant orientées en oblique par rapport à la face frontale, et ledit autopiqueur (40) et ladite lancette (1) étant réalisés de telle sorte que pour l'examen optique d'un échantillon de liquide corporel, mouillant la surface de mouillage (34), dans une lancette (1) insérée dans l'autopiqueur, une lumière émise par la source lumineuse (21) parvient sur la surface oblique (33) de l'élément transparent (30) de la lancette (1) insérée et est guidée par réflexion totale vers le détecteur (28) en passant à travers une interface entre la surface de mouillage (34) et l'échantillon de liquide corporel, mouillant la surface de mouillage (34).
